# EUROPEAN PATENT SPECIFICATION

(11) **EP 1 535 999 B1**
(45) Date of publication and mention of the grant of the patent: **19.12.2007**
(21) Application number: 03738618.2
(22) Date of filing: 01.07.2003
(51) Int. Cl.: C12N 9/52, A23C 19/032

(54) **MILK-COAGULATING ENZYME ORIGINATING IN BACTERIUM AND PROCESS FOR PRODUCING CHEESE USING THE SAME**
AUS EINEM BAKTERIUM STAMMENDES MILCHGERINNUNGSENZYM UND VERFAHREN ZUR HERSTELLUNG VON KÄSE UNTER VERWENDUNG DAVON
ENZYME DE COAGULATION DU LAIT PRODUITE PAR UNE BACTERIE ET PROCEDE CORRESPONDANT DE FABRICATION DE FROMAGE

(30) Priority: 02.07.2002 JP 2002194016
(43) Date of publication of application: 01.06.2005
(73) Proprietor: Mahoroba Co., Ltd, Sapporo-shi, Hokkaido 063-0035 (JP)
(72) Inventor: YASOKAWA, Daisuke Hokkaido Food Processing Res.Cnt, Ebetsu-shi, Hokkaido 069-0836 (JP); SAWADA, Hitoshi Hokkaido Food Processing Res.Cntr, Ebetsu-shi, Hokkaido 069-0836 (JP); NAKAGAWA, Ryoji Hokkaido Food Processing Res.Cntr, Ebetsu-shi, Hokkaido 069-0836 (JP); KAWAKAMI, Makoto Hokkaido Food Processing Res.Cntr, Ebetsu-shi, Hokkaido 069-0836 (JP); NAGASHIMA, Koji Hokkaido Food Processing Res.Cntr., Ebetsu-shi, Hokkaido 069-0836 (JP); MIYASHITA, Shuhei c/o MAHOROBA CO., LTD., Sapporo-shi, Hokkaido 063-0035 (JP); MIYASHITA, Hiroko c/o MAHOROBA CO., LTD., Sapporo-shi, Hokkaido 063-0035 (JP)
(74) Representative: Ebner von Eschenbach, Jennifer
(86) International application number: PCT/JP2003/008373
(87) International publication number: WO 2004/005503

(56) References cited:
- WO-A-93/14117
- WO-A-94/01532
- WO-A-02/082916
- WO-A1-00/73429
- US-A- 3 661 594
- MATOS J ET AL: "Genetic characterization of pepP, which encodes an aminopeptidase P whose deficiency does not affect Lactococcus lactis growth in milk, unlike deficiency of the X-prolyl dipeptidyl aminopeptidase" APPLIED AND ENVIRONMENTAL MICROBIOLOGY, vol. 64, no. 11, November 1998 (1998-11), pages 4591-4595, XP002358467 ISSN: 0099-2240
- EMMONS D B ET AL: "MILK-CLOTTING ENZYMES 1. PROTEOLYSIS DURING CHEESE MAKING IN RELATION TO ESTIMATED LOSSES OF YIELD" JOURNAL OF DAIRY SCIENCE, vol. 73, no. 8, 1990, pages 2007-2015, XP002358468 ISSN: 0022-0302
- FIAT A-M ET AL: "CASEINS OF VARIOUS ORIGINS AND BIOLOGICALLY ACTIVE CASEIN PEPTIDES AND OLIGOSACCHARIDES STRUCTURAL AND PHYSIOLOGICAL ASPECTS" MOLECULAR AND CELLULAR BIOCHEMISTRY, vol. 87, no. 1, 1989, pages 5-30, XP008057257 ISSN: 0300-8177
- DAISUKE YASOGAWA ET AL.: 'Paenibaciluls-zoku saikin no seisan suru gyonyu koso' JAPAN SOCIETY FOR BIOSCIENCE, BIOTECHNOLOGY AND AGROCHEMISTRY TAIKAI YOSHISHU (2A10P07) 2003, page 34, XP002974166

## Description

### TECHNICAL FIELD

The present invention relates to a milk-coagulating enzyme of bacterial origin and a method for producing cheese using the same, and more particularly to a milk-coagulating enzyme produced by a bacterium of the genus *Paenibacillus* and a method for producing cheese and a cheese-like food product using the same. The present invention is useful in the provision of a method for producing a new type of cheese and a cheese-like food product characterized by having a good aroma, flavor, and texture, using a novel milk-coagulating enzyme of bacterial origin.

### BACKGROUND ART

Conventionally, a milk-coagulating enzyme is required during cheese production to coagulate the milk raw materials. Traditionally, chymosin has been extracted from the fourth stomach of preweaned calves and prepared for use as the milk-coagulating enzyme. However, since the early 1970s, many researchers have been actively engaged in the research and development of a replacement milk-coagulating enzyme for chymosin, due to a chronic shortage of calves to provide the chymosin raw materials.

To date, numerous milk-coagulating enzymes of animal, plant, and microbial origin have been identified as replacements for chymosin and trialed in cheese production. However, the only milk-coagulating enzymes to be applied in practice as replacements for chymosin are pepsin of animal origin and microbial rennet originating from three types of filamentous fungi: *Endothia parasitica, Mucor pusillus,* and *Mucor* miehei.

U.S. 3,661,594 discloses the production of a milk coagulating enzyme by a process comprising culturing a strain of *Bacillus polymyxa*. This milk coagulating enzyme is useful for making cheese.

### DISCLOSURE OF THE INVENTION

Amid the situation described above, the present inventors conducted thorough investigations in a bid to discover a new milk-coagulating enzyme of microbial origin that would be useful in the development of a new type of cheese characterized by having a good aroma, taste, and texture. The present inventors determined that an isolated bacterial culture supernatant exhibited potent milk-coagulating activity and completed development of the present invention through further research.

Thus, an object of the present invention is to provide a production method for cheese and a cheese-like food product using a milk-coagulating enzyme produced by a bacterium of the genus *Paenibacillus.*

A further object of the present invention is to provide the novel milk-coagulating enzyme of bacterial origin used in the production of the above foods.

To resolve the problems described above, the present invention is constituted from the following technical means.
(1) An milk-coagulating enzyme exhibiting milk-coagulating activity that is produced by a *Paenibacillus* sp. and has the following enzymatic properties: milk-coagulating enzyme characterized by (a) function: having an activity of coagulating milk to form a curd; (b) substrate specificity: acting on κ-casein as a substrate and specifically cleaving Thr94-Met95 in the presence of calcium; (c) optimum pH: 6.0-7.0; and (d) molecular weight: 35,000-37,000 Da when measured by SDS-PAGE.
(2) The enzyme according to (1) above that is salt precipitated in 50-80% saturated ammonium sulfate solution.
(3) The enzyme according to (2) above that is characterized by being salt precipitated in 50-80% saturated ammonium sulfate solution, having a stable pH range of 6.0-8.0, and having a stable temperature range of 40-50°C.
(4) The enzyme according to (1) above that is produced by *Paenibacillus* sp. accession number FERM P-18138.
(5) A method of cheese production that is characterized by the formation of a curd using the milk-coagulating enzyme according to any of (1) through (4) above, whey separation, and subsequent ripening during the production of cheese using milk raw materials.

Following is a more detailed description of the present invention.

The present invention is characterized in that a specific bacterium capable of producing a milk-coagulating enzyme is cultured and the milk-coagulating enzyme in the present invention is extracted from the culture obtained, and in that it provides for the production of cheese and a cheese-like food product using the milk-coagulating enzyme in the present invention. In the present invention, the bacterial strain that produces the above milk-coagulating enzyme is classified as belonging to the genus *Paenibacillus* according to its physiological characteristics and a comparison of the nucleotide sequence of the 16S ribosomal RNA gene. This bacterial strain has been deposited at the International Patent Organism Depositary of the National Institute of Advanced Industrial Science and Technology as accession number FERM P-18138, identification of microorganism Paeni.

The method for obtaining the milk-coagulating enzyme from the *Paenibacillus* sp. culture can comprise, for example when the above bacterial strain is cultured on a solid medium, the addition of an equivalent amount of water or buffer solution to the culture and extraction to obtain a crude solution of the milk-coagulating enzyme, and the condensing and drying of a suitable amount of the crude enzyme solution to obtain a crude milk-coagulating enzyme powder. Further, when the above bacterial strain is cultured in solution, the method for obtaining the milk-coagulating enzyme can comprise removal of the bacteria from the solution by centrifugation, precipitation of the milk-coagulating enzyme by the addition to the culture supernatant of a substance such as aluminum sulfate or sodium sulfate or an organic solvent such as acetone or alcohol, and the separation, recovery, and drying of the precipitate to produce a crude milk-coagulating enzyme powder. However, the present invention is not limited by these methods and can be used with any suitable method.

Following are the standard enzyme purification methods that can be used as the purification method for the milk-coagulating enzyme in the present invention. Ideally, for example, the crude enzyme solution obtained from the culture supernatant of the *Paenibacillus* sp. culture solution can be purified using hydrophobic column chromatography and cation-exchange column chromatography methods. However, the present invention is not limited by these methods. In terms of culture methods for the *Paenibacillus* sp. used in the present invention, with culture on solid media, for example, the method can be exemplified by using a standard agar medium (0.25% yeast extract, 0.5% peptin, 0.1% glucose, 1.5% agar) that has had skim milk added to make 1 w/v%, culturing overnight at 35°C, and then culturing at room temperature, while with culture in solution, for example, the method can be exemplified by using some variation on a liquid culture medium (basic liquid culture medium) with a basic composition of 0.5% corn steep liquor, 0.5% soluble starch, and 0.5% NaH₂PO₄ (pH 7.0) and culturing on a rotating shaker at 180 rpm and 37°C. However, the present invention is not limited by these methods and can be used with any suitable method.

The milk-coagulating enzyme in the present invention can be obtained as a purified enzyme preparation by extracting the crude enzyme solution from the culture and, ideally, after ammonium sulfate salt precipitation using 50-80% saturated ammonium sulfate solution, purifying using cation-exchange chromatography and hydrophobic chromatography.

Thus, fractionation and purification methods for the milk-coagulating enzyme can be exemplified by, for example, culturing the above bacterial strain for 8 hours in a basic liquid culture medium, harvesting the bacteria, resuspending in 0.5% starch-5 mM CaCl₂, culturing overnight, removing the bacteria and other particulate matter by centrifugation, dissolving aluminum sulfate in the supernatant to 50% saturation, removing the polysaccharides including starch by ultracentrifugation, adding aluminum sulfate to the supernatant obtained to 80% saturation, dissolving, leaving overnight at 4°C, recovering the precipitate by centrifugation, dissolving in a buffer solution of 3.3 mM MES/3.3 mM HEPES/3.3 mM sodium acetate-5 mM CaCl₂ (pH 5.5), passing through a filter with a pore size of 0.8 pm, overnight dialysis against the same buffer solution, subjecting the enzyme preparation obtained to cation-exchange column chromatography, isolating the active fraction, adding aluminum sulfate to the active fraction to a final concentration of 1M aluminum sulfate, and conducting hydrophobic chromatography to isolate the active fraction. The active fraction obtained with the above method is identified as an almost single band following 10% SDS-PAGE. The present invention is not limited by the above methods for the fractionation and purification of the milk-coagulating enzyme and suitable methods and conditions can be determined in accordance with the above methods.

Following are the enzymological properties of the milk-coagulating enzyme in the present invention.
(1) Optimum pH and stable pH
   The optimum pH and pH stability of the milk-coagulating enzyme in the present invention were investigated. The optimum pH was measured using 100 mM ionic strength buffer solution adjusted to a fixed pH to which was added skim milk and calcium chloride such that the solution was 10% w/v skim milk-10 mM CaCl₂. For pH stability, the milk-coagulating enzyme was placed for 10 minutes at 35°C in a 50 mM ionic strength buffer solution adjusted to a fixed pH and the residual activity was compared. The results showed that maximum milk-coagulating activity was achieved at pH 6.0-7.0, with activity at pH 8.0 approximately 64% of that at pH 7.0. Further, milk-coagulating activity was stable at pH 6.0-8.0, but was almost completely lost at pH 4.0.
(2) Optimal working temperature and thermal stability
   Following are investigations into the optimal working temperature and thermal stability of the milk-coagulating enzyme in the present invention. The optimal working temperature was measured using milk-coagulating activity at fixed temperatures. For thermal stability, the milk-coagulating enzyme was placed for 10 minutes at fixed temperatures in 20 mM MES/HEPES/sodium acetate-5mM CaCl₂ and the residual activity was compared. The results showed that this enzyme is stable up to 40-50°C and milk-coagulating activity increased as the temperature increased, but began to decline as the temperature approached 50°C and was almost completely lost at 70°C.
(3) Inhibitors
   The milk-coagulating activity of the milk-coagulating enzyme in the present invention was investigated using inhibitors. The milk-coagulating activity was inhibited by EDTA, while Ca ions were vital for activity expression and maintenance.
(4) Molecular weight
   The molecular weight of the milk-coagulating enzyme in the present invention was approximately 35,000-37,000 Da when measured by SDS-PAGE.
(5) Activity assay methods
   The milk-coagulating activity of the milk-coagulating enzyme in the present invention was investigated in accordance with the method by Arima et al. That is, using a 10% reduced fat milk dissolved in 0.01 M calcium chloride as the base, 0.5 mL enzyme solution at a concentration adjusted to allow curd fragment formation after 5-10 minutes was added to 5 mL base and left at 35°C. The solution was agitated from time to time and curd fragment formation was observed. The time to curd fragment formation was measured and the amount of enzyme required to start milk coagulation after 1 minute was defined as 400 units.
(6) Action
   The enzyme in the present invention exhibits milk-coagulating activity such that it coagulates milk and forms a curd.
(7) Substrate specificity: κ-casein was dissolved at a concentration of 10 mg/mL in 20 mM MOSO (pH 6.5)-10 mM CaCl₂, 1/20th the volume of milk-coagulating enzyme solution was added, the resulting solution was incubated for 15 minutes at 37°C, the digestion products were isolated with Tris/Tricine-buffered SDS-PAGE and blotted onto a PVDF membrane, and the N-terminal amino acid sequence was analyzed. For amino acid sequencing, after dissolving the milk-coagulating enzyme in 0.1% TFA, this same solution was added to an equilibrated Phenyl 5PW RP column and eluted using an acetonitrile concentration gradient. The main peak N-terminal amino acid sequence was analyzed using a protein sequencer. The results showed that the milk-coagulating enzyme specifically cleaved the κ-casein substrate at Thr94-Met95 in the presence of calcium.

With the present invention, cheese and a cheese-like food product can be made through a process to produce cheese using milk raw materials, by the formation of a curd using the above milk-coagulating enzyme, whey separation, and subsequent ripening. In terms of the specific processes in the present invention for the production of the cheese and the cheese-like food product, in addition to the use of the above milk-coagulating enzyme, normal cheese production methods can be applied as exemplified by, for example, heat sterilization of the raw milk at 65°C; rapid cooling at 30°C; addition of the milk-coagulating enzyme in the present invention, lactic-acid bacteria starter culture, and calcium chloride according to established methods; maintenance under temperature-controlled conditions; cutting of the curd that has formed and shaking; extraction of the whey by separating from the curd with gauze; pressing and soaking in 20% brine; drying; vacuum packaging; and storage in a ripening chamber at 15°C to mature. However, these processes and conditions are not limited by the above methods and can be set as appropriate in accordance with the type of product.

The cheese and the cheese-like food product produced using the methods in the present invention were investigated in terms of shearing stress using a piano wire, food texture, electron microscope observation of the cheese in cross section, and free amino acid composition, and were compared with cheese produced under the same conditions using normal calf rennet. The results showed that that the characteristics of the two cheeses were almost equivalent, apart from the cheese in the present invention being superior to rennet cheese in terms of shearing stress and the contents of the amino acid glutamic acid, which imparts flavor, and the hydrophobic amino acid isoleucine, and the cheese in the present invention having a slightly crumbly texture, and showed that the cheese and the cheese-like food product in the present invention were of an equivalent quality to the rennet cheese product.

### BRIEF DESCRIPTION OF THE DRAWINGS

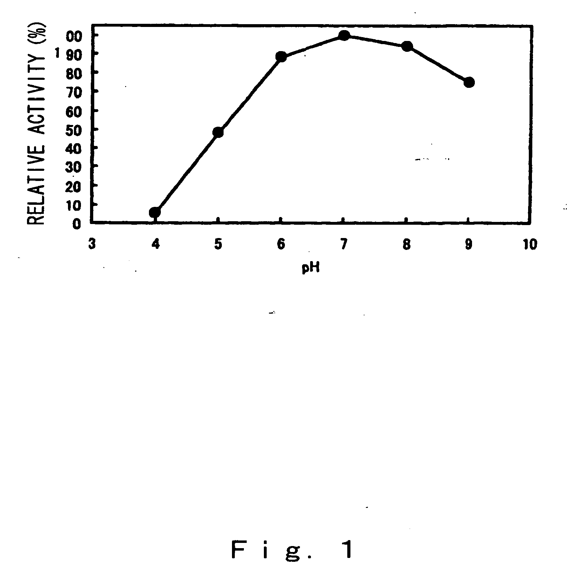

FIG. 1 shows the pH stability of the milk-coagulating enzyme according to the present invention;

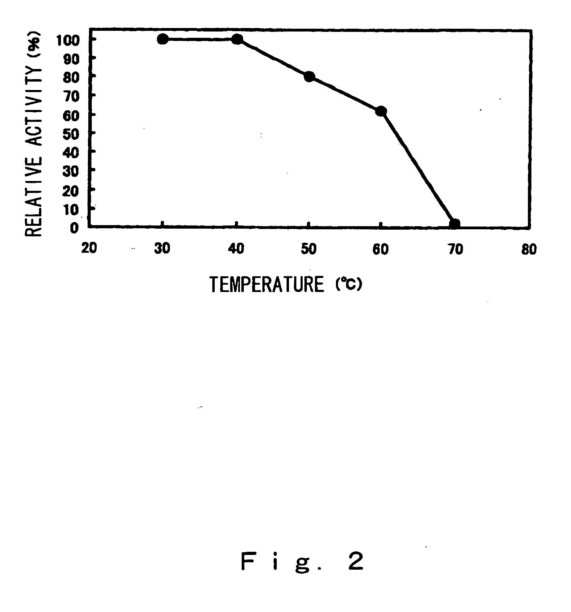

FIG. 2 shows the temperature stability of the milk-coagulating enzyme according to the present invention; and

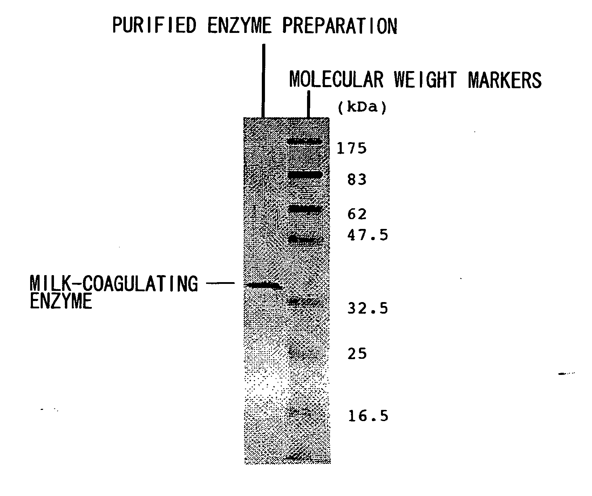

FIG. 3 shows the results of 10% polyacrylamide gel electrophoresis for the purified milk-coagulating enzyme according to the present invention.

### BEST MODE FOR CARRYING OUT THE INVENTION

Following is a concrete description of the present invention through examples; however, the present invention is not limited whatsoever by the following examples.

### Example 1

The *Paenibacillus* sp. bacterial strain (FERM P-18138) was cultured at 37°C until the end of the log phase and 1.2 L of the culture solution was centrifuged to harvest the bacteria. The bacteria were resuspended in 600 mL 5 mM aqueous calcium chloride containing 0.5% soluble starch and cultured overnight. After culturing, the culture solution was centrifuged at 4°C to obtain a crude enzyme solution. Aluminum sulfate was added to the crude enzyme solution to 50% saturation, the precipitate was removed by centrifugation, and aluminum sulfate was added to the supernatant obtained to 80% saturation. The precipitate was recovered by centrifugation and dialysis was performed overnight against 3.3 mM MES/3.3 mM HEPES/3.3 mM sodium acetate-5 mM calcium chloride (pH 5.5). The result was that a BSA standard of approximately 2.4 mg of enzyme preparation was obtained with a milk-coagulating activity of approximately 1,100,000 units.

### Example 2

The enzyme preparation obtained in Example 1 was loaded into a Poros HS 20 micron column (4.6 mm in diameter x 100 mm in height) equilibrated with a buffer solution (buffer solution A) of 3.3 mM MES/3.3 mM HEPES/3.3 mM sodium acetate containing 5 mM calcium chloride (pH 5.5) and eluted under a linear gradient with buffer solution A containing 0-0.2 M salt to recover the active fraction. Aluminum sulfate was added to the active fraction to a final concentration of 1M and this was loaded into a Poros HP2 20 micron column (4.6 mm in diameter x 100 mm in height) equilibrated with a buffer solution (buffer solution B) of 15 mM tris-hydroxymethyl aminomethane/15 mM bis-tris propane containing 1M aluminum sulfate and 5 mM calcium chloride (pH 7.5) and eluted under a linear gradient with buffer solution B containing 1M-200 mM aluminum sulfate to recover the active fraction. Dialysis of the active fraction was performed against a buffer solution of 5 mM tris-hydroxymethyl aminomethane/5 mM bis-tris propane containing 5 mM calcium chloride (pH 7.0), and a BSA standard of approximately 250 µg of purified enzyme preparation was obtained. This enzyme preparation was purified 1.31-fold using this two-step column chromatography procedure, with a recovery rate of 13.6%.

### Example 3

### (1) Cheese production

The *Paenibacillus* sp. bacteria were spread onto agar in 100 Petri dishes 9 cm in diameter and propagated at 37°C, the milk-coagulating enzyme was extracted from the agar with water, and the bacteria and agar fragments were removed by centrifugation. After filtration, the milk-coagulating enzyme was suspended in skim milk to 0.5% w/v and freeze-dried to make a milk-coagulating enzyme preparation. Milk was heat sterilized for 30 minutes at 65°C, cooled to 30°C, and inoculated with 1% lactic-acid bacteria starter culture, to which was added this milk-coagulating enzyme preparation dissolved in a small amount of water. Thereafter, established methods were used for cutting, whey off, pressing, and salting, with 6 months ripening to produce Gouda cheese.

### (2) Results

Table 1 shows the free amino acid contents for the cheese obtained from the above production process using the milk-coagulating enzyme in the present invention as rennet (present invention sample) and for the cheese obtained using calf rennet (comparator sample). Further, the shearing stress obtained using a piano wire and a rheometer was 231.8±19.8 gf for the present invention sample and 207.8 ± 28.1 gf for the comparator sample.

**Table 1**

| Free amino acid content (mg/g) | | |
|---|---|---|
| Free amino acids | Comparator sample | Present invention sample |
| Asp | 0.20 | 0.19 |
| Thr | 0.45 | 0.34 |
| Ser | 0.33 | 0.31 |
| Asn | 0.52 | 0.83 |
| Glu | 2.06 | 2.39 |
| Gln | 0.06 | 0.07 |
| Gly | 0.17 | 0.25 |
| Ala | 0.23 | 0.21 |
| Val | 0.53 | 0.78 |
| Met | 0.33 | 0.21 |
| Ile | 0.7 | 0.37 |
| Leu | 1.49 | 1.78 |
| Tyr | 0.44 | 0.49 |
| Phe | 0.82 | 0.86 |
| Trp | 0.19 | nd |
| Lys | 0.89 | 0.98 |
| His | 0.27 | 0.21 |
| Arg | 0.28 | 0.13 |
| Pro | 0.21 | 0.32 |
| Total | 10.17 | 10.72 |

### Example 4

### (Cheese production)

The *Paenibacillus* sp. bacteria were spread onto 1% skim milk-added standard agar in 100 Petri dishes, cultured overnight at 35°C, and cultured for 1 day at room temperature. The enzyme was extracted from the agar with 1.6 L water and the bacteria and agar fragments were removed by centrifugation. After filtration, skim milk was added to make 0.5% (w/v) in 1.3 L and the solution was freeze-dried. Approximately 10 L of raw milk was heat sterilized for 30 minutes at 65°C, rapidly cooled to 30°C, and after freeze drying, established methods were followed for the addition of calcium chloride, lactic-acid bacteria starter culture, and the entire enzyme extract solution. Commercially available calf rennet was used as a comparator. After leaving under temperature-controlled conditions for 1 hour, the curd that had formed was cut and shaken for 30 minutes. The whey was separated from the curd using gauze, followed by pressing and soaking in 20% brine, drying, vacuum packaging, and storage in a ripening chamber at 15°C.

The mass of cheese produced was 1,333.3 g when the milk-coagulating enzyme in the present invention was used and 1,226.1 g when calf rennet was used. The cheese produced using the milk-coagulating enzyme in the present invention was slightly more crumbly in texture. Electron microscopy of the cheeses in cross section showed no major visible differences in appearance.

The free amino acid composition of the cheeses were compared after 6 months' ripening. The results showed that the cheese produced using the milk-coagulating enzyme in the present invention tended to have a slightly higher glutamic acid content, which is an amino acid that imparts flavor. In terms of hydrophobic amino acids (valine, leucine, isoleucine), the cheese produced using the milk-coagulating enzyme in the present invention had almost double the isoleucine content, but the total content of these three amino acids was roughly the same as the comparator cheese.

### Industrial Applicability

As described in detail above, the present invention relates to a milk-coagulating enzyme of bacterial origin and a process for producing cheese using the same. According to the present invention, the following remarkable effects are attained: 1) a new milk-coagulating enzyme can be provided; 2) the milk-coagulating enzyme in the present invention can be used as a replacement enzyme for chymosin in the production of cheese and a cheese-like food product; 3) a production method for a new cheese and a cheese-like food product using the above milk-coagulating enzyme can be provided; and 4) according to the above method, cheese and a cheese-like food product of an equivalent quality to rennet cheese can be provided.

### Reference for the microorganism deposit

Name of the depositary organization: International Patent Organism Depositary, National Institute of Advanced Industrial Science and Technology
Address: Central 6, Higashi 1-1-1, Tsukuba City, Ibaraki Prefecture, Japan 305-8566
Date of deposit: 8 December 2000
Accession number: FERM P-18138
Microorganism indicator: Paeni

## Claims

1. An milk-coagulating enzyme exhibiting milk-coagulating activity that is produced by a bacterium of the genus *Paenibacillus* and **characterized by** having the following enzymatic properties:
(1) function: having an activity of coagulating milk to form a curd;
(2) substrate specificity: acting on κ-casein as a substrate and specifically cleaving Thr94-Met95 in the presence of calcium;
(3) optimum pH: 6.0-7.0; and
(4) molecular weight: 35,000-37,000 Da when measured by SDS-PAGE.

2. The enzyme according to claim 1, wherein the enzyme is salt precipitated in 50-80% saturated ammonium sulfate solution.

3. The enzyme according to claim 2, wherein the enzyme is **characterized by** being salt precipitated in 50-80% saturated ammonium sulfate solution, having a stable pH range of 6.0-8.0, and having a stable temperature range of 40-50°C.

4. The enzyme according to claim 1, wherein the enzyme is produced by *Paenibacillus* sp. accession number FERM P-18138.

5. A method of producing cheese using milk as raw material, that is **characterized by** the formation of a curd using the milk-coagulating enzyme according to any of claims 1 through 4, whey separation, and subsequent ripening.

## Patentansprüche

1. Milchgerinnungsenzym mit Milchgerinnungsaktivität, das mit Hilfe eines Bakteriums des Genus *Paenibacillus* erzeugt wird und mit den folgenden enzymatischen Eigenschaften **gekennzeichnet** ist:
(1) Funktion: es verfügt über eine Aktivität zum Koagulieren von Milch unter Erzeugung von Käsebruch;
(2) Substratspezifität: wirkt auf κ-Casein als ein Substrat und spaltet speziell Thr94-Met95 in Gegenwart von Calcium;
(3) optimaler pH-Wert: 6,0 bis 7,0 und
(4) Molekulargewicht: 35.000 bis 37.000 Da bei Messung mit Hilfe der SDS-Polyacrylamidgelelektrophorese.

2. Enzym nach Anspruch 1, wobei das Enzym salzgefällt ist in 50% bis 80% gesättigter Ammoniumsulfat-Lösung.

3. Enzym nach Anspruch 2, wobei das Enzym **dadurch gekennzeichnet ist, dass** es in 50% bis 80% gesättigter Ammoniumsulfat-Lösung salzgefällt ist mit einem stabilen pH-Wertbereich von 6,0 bis 8,0 und das einen stabilen Temperaturbereich von 40° bis 50°C hat.

4. Enzym nach Anspruch 1, wobei das Enzym erzeugt wird durch *Paenibacillus spec.* mit der Hinterlegungsnummer FERM P-18138.

5. Verfahren zum Herstellen von Käse unter Verwendung von Milch als Ausgangsmaterial **gekennzeichnet durch** die Erzeugung eines Käsebruchs unter Verwendung des Milchgerinnungsenzyms nach Anspruch 1 bis 4, **durch** Molkeabtrennung und **durch** anschließende Reifung.

## Revendications

1. Enzyme faisant coaguler le lait présentant une activité de coagulation du lait, laquelle est produite par une bactérie du genre *Paenibacillus* et **caractérisée en ce qu'**elle a les propriétés enzymatiques suivantes :
(1) fonction : ayant une activité de coagulation du lait pour former un caillé ;
(2) spécificité vis-à-vis du substrat : agissant sur la κ-caséine en tant que substrat et clivant spécifiquement la liaison Thr94-Met95 en présence de calcium ;
(3) pH optimal : 6,0-7,0 ; et
(4) masse moléculaire : 35 000-37 000 Da lorsqu'elle est mesurée par SDS-PAGE.

2. Enzyme selon la revendication 1, ladite enzyme étant précipitée par un sel dans une solution de sulfate d'ammonium saturée à 50-80 %.

3. Enzyme selon la revendication 2, ladite enzyme étant **caractérisée en ce qu'**elle est précipitée par un sel dans une solution de sulfate d'ammonium saturée à 50-80 %, **en ce qu'**elle a une plage de pH dans laquelle elle est stable de 6,0-8,0 et **en ce qu'**elle a une plage de températures dans laquelle elle est stable de 40-50°C.

4. Enzyme selon la revendication 1, ladite enzyme étant produite par le microorganisme de l'espèce *Paenibacillus sp.* ayant le numéro d'enregistrement FERM P-18138.

5. Procédé de production de fromage utilisant du lait en tant que matière première, lequel est **caractérisé par** la formation d'un caillé en utilisant l'enzyme faisant coaguler le lait selon l'une quelconque des revendications 1 à 4, la séparation du lactosérum et l'affinage subséquent.
